# EUROPÄISCHE PATENTSCHRIFT

(11) **EP 1 211 991 B1**
(45) Veröffentlichungstag und Bekanntmachung des Hinweises auf die Patenterteilung: **27.10.2004**
(21) Anmeldenummer: 00908899.8
(22) Anmeldetag: 21.03.2000
(51) Int. Cl.: A61B 17/58

(54) **REPOSITIONSVORRICHTUNG FÜR KNOCHENFRAGMENTE**
REPOSITIONING DEVICE FOR BONE FRAGMENTS
DISPOSITIF DE REDUCTION POUR FRAGMENTS OSSEUX

(30) Priorität: 15.09.1999 DE 29916202 U
(43) Veröffentlichungstag der Anmeldung: 12.06.2002
(73) Patentinhaber: SYNTHES AG Chur, 7002 Chur (CH)
(72) Erfinder: HEHLI, Markus, CH-7276 Davos-Frauenkirch (CH); RUPP, Stephan, CH-7270 Davos-Platz (CH); SCHWYN, Ronald, CH-7277 Davos-Glaris (CH); MESSMER, Peter, CH-4104 Oberwil (CH); REGAZZONI, Pietro, CH-4054 Basel (CH)
(74) Vertreter: Lusuardi, Werther Giovanni, Dr.
(86) Internationale Anmeldenummer: PCT/CH2000/000162
(87) Internationale Veröffentlichungsnummer: WO 2001/019265

(56) Entgegenhaltungen:
- CH-A- 537 735
- CH-A- 680 561
- DE-A- 3 151 144
- DE-A- 19 618 354
- DE-U- 8 526 619
- DE-U- 8 536 668
- DE-U- 29 610 638
- US-A- 5 601 550

## Beschreibung

Die Erfindung bezieht sich auf eine Vorrichtung zum Reponieren von Knochenfragmenten, insbesondere an Röhrenknochen oder am Becken gemäss dem Oberbegriff des Patentanspruchs 1.

Zur Anwendung einer minimal invasiven Chirurgie in der Traumatologie und Orthopädie sind oft solche Repositionsinstrumente oder -vorrichtungen notwendig, welche nur enge Zugänge am menschlichen oder tierischen Körper erfordern. Sowohl die Reposition als auch das Halten der Reposition ist oft problematisch. Die meisten Repositionszangen funktionieren nach einem Scherenmechanismus, indem zwei sich kreuzende und durch ein Gelenk verbundene Hebel X-förmig und damit grossflächig in einer Ebene geöffnet und geschlossen werden. Insbesondere bei Frakturen in der Tiefe des Beckens verhindern die Weichteile und der schlitzförmige Zugang oft das Setzen einer solchen Klemme senkrecht zur Frakturebene. Klemmvorrichtungen mit axialem Verschlussmechanismus sind daher oft vorteilhaft.

Eine Notfallbeckenzwinge mit geradlinig in einer Achse verschiebbaren Spannelementen ist aus der CH 680 561 GANZ bekannt. Diese bekannte Beckenzwinge besteht aus einer longitudinalen Schiene und zwei auf der Schiene bewegbare Armen. Die Arme besitzen für den Knochenkontakt bestimmte Spitzen, welche sich mittels Gewindespindeln bewegen lassen und zur Krafteinleitung bei der Reponierung des Beckenknochens anwendbar sind. Allerdings besteht die Gefahr, dass bei schräg zur Spannrichtung stehenden Knochenoberflächen die Spitzen auf den Knochenoberflächen abgleiten.

Eine chirurgische Operationsvorrichtung mit einer Linearführung, einem ersten darauf verschiebbaren Halter und einer zweiten Führung in Form eines Gradbogens, worauf ein zweiter Halter verschiebbar angeordnet ist, ist aus der DE 196 18 354 KÖNIGSBERGER bekannt. Die beiden Halter wirken als Fixationselemente für die gebrochenen Knochenfragmente.

Eine weitere Vorrichtung zum Zusammenfügen von Knochenteilen mit einem Haltekörper, einem Haltestück und einem mit dem Haltekörper lösbar verbunden Haltebügel ist aus der DE 85 36 668 BAUER bekannt.

Eine Lehre zur Durchführung einer Verschraubung von Knochenfrakturen mit einem Bügel und einer auf dem Bügel verschiebbaren Spannbacke ist aus der CH 537 735 bekannt.

Hier will die Erfindung Abhilfe schaffen. Der Erfindung liegt die Aufgabe zugrunde, eine Repositionsvorrichtung zu schaffen, weiche einen axialen Verschlussmechanismus aufweist und erhöhte Sicherheit gegen Abgleiten der Spannelemente beim Aufbringen der zur Reposition notwendigen Kraft bietet.

Die Erfindung löst die gestellte Aufgabe mit einer Vorrichtung zum Reponieren von Knochenfragmenten, insbesondere an Röhrenknochen oder am Becken, welche die Merkmale des Anspruchs 1 aufweist.

Die Vorrichtung zum Reponieren von Knochenfragmenten, umfasst ein erstes und ein zweites Spannelement, welche mittels eines Verbindungselementes so verbunden werden, dass das zweite Spannelement geradlinig gegenüber dem ersten Spannelement verschiebbar ist. Damit die Vorrichtung um einen Knochen herumgeführt werden kann, ist das erste Spannelement mit einem Bügel, dessen Enden in der Längsachse des zweiten Spannelementes liegen, versehen, so dass ein axialer Klemmmechanismus entsteht, welcher durch ein Spannmittel blockierbar ist. Am andern Ende des Bügels ist ein zur Verschiebungsachse paralleler, mit dem Verbindungselement verbundener Stab angeordnet. Ebenfalls umfasst die Vorrichtung einen Handgriff. Das zweite Spannelement ist prismatisch oder zylindrisch ausgebildet und so angeordnet, dass das vordere Ende zur Anlage an ein weiteres Knochenfragment dient. Die Spannmittel ermöglichen, dass eine axiale Druckkraft auf Knochenfragmente, welche zwischen den Spannelementen eingespannt sind, ausübbar ist und die Spannelemente relativ zueinander lösbar feststellbar sind.

In einer bevorzugten Ausführungsform der erfindungsgemässen Vorrichtung umfasst das erste Spannelement eine am vorderen Endstück angeordnete, gegen das Innere des Bügels gerichtete, pyramiden- oder kegelförmige Erhebung, welche zur Anlage an ein Knochenfragment dient. Die Erhebung am vorderen Ende des Bügels weist eine Spitze auf, welche in die Oberfläche eines Knochens einpressbar ist und ebenfalls zur Verringerung der Gefahr eines Abgleitens an einer schräg zur Längsachse stehenden Knochenoberfläche dient. Ferner ist das vordere Ende des zweiten Spannelementes kronenförmig mit stirnseitigen Zacken, welche in die Oberfläche eines Knochens einpressbar sind, ausgebildet. Dadurch lässt sich die Gefahr eines Abgleitens an einer schräg zur Längsachse stehenden Knochenoberfläche verringern.

In wiederum einer weiteren Ausführungsform der erfindungsgemässen Vorrichtung umfasst das vordere Endstück des ersten Spannelementes konzentrisch zur Längsachse eine Bohrung mit einem Innengewinde und eine Spannschraube, welche von der Aussenseite des Bügels in das Innengewinde schraubbar ist und gegen die Innenseite des Bügels gerichtet ein vorderes Ende mit Mitteln zur Anlage an einen Knochen umfasst. Anstelle der einschraubbaren Spannschraube sind auch Einsätze möglich, welche mittels einer federnden Arretierung oder eines Bajonettverschlusses axial verriegelbar sind. Vorteilhaft an diesen Ausführungsformen ist, dass der Bügel um die Weichteile herum geführt werden kann und nur die Spannschraube oder der Einsatz durch eine Stichinzision durch den Weichteilmantel zum Knochen geführt werden muss.

Die Mittel zur Anlage an einen Knochen sind als zur Längsachse koaxiale, kegel- oder pyramidenförmige Spitzen, als zur Längsachse koaxiale Kugel mit einer ebenfalls zur Längsachse koaxialen, kegel- oder pyramidenförmigen Spitze, als mittels einem Kugelgelenk mit dem vorderen Ende verbindbarem Aufsatz mit kegel- oder pyramidenförmiger Spitze oder kronenförmig mit stirnseitigen Zacken ausführbar.

In einer weiteren Ausführungsform der erfindungsgemässen Vorrichtung sind das vordere Endstück des ersten Spannelementes und dass zweite Spannelement konzentrisch zur Längsachse mit Bohrungen versehen, welche die Aufnahme eines Führungsdrahtes resp. Kirschnerdrahtes koaxial zur Längsachse gestatten. Bei den Ausführungsformen des vorderen Endstückes mit Spannschraube oder Einsatz ist zur Aufnahme des Führungsdrahtes respektive Kirschnerdrahtes die Spannschraube oder der Einsatz konzentrisch zur Längsachse durchbohrt.

Diese Bohrungen können in anderen Ausführungsformen der erfindungsgemässen Vorrichtung am ersten und/oder zweiten Spannelement auch einen solchen Durchmesser aufweisen, dass eine Knochenschraube durch die Bohrung hindurch zur Knochenoberfläche geschoben und in den Knochen eingeschraubt werden kann. Ebenfalls möglich ist die Ausgestaltung einer Bohrung, deren Durchmesser die Aufnahme eines Trocars gestattet. Der Trocar dient als Platzhalter, so dass beim Einbringen des zweiten Spannelementes, der Spannschraube oder des Einsatzes durch die Stichinzision die Weichteile geschont werden und nicht durch das hohle Spannelement verletzt werden. Nach dem Einbringen der Vorrichtung durch die Weichteile wird der Trocar aus der Vorrichtung entfernt und nach Bedarf Kirschnerdrähte respektive Führungsdrähte oder Knochenschrauben in den Knochen eingebracht.

In einer anderen Ausführungsform der erfindungsgemässen Vorrichtung ist das vordere Ende des zweiten Spannelementes mit Mittel zum Festklemmen einer Knochenplatte ausgestattet. Diese Mittel bestehen vorzugsweise aus elastischen Zungen, welche zur Längsachse parallel angeordnet sind und in radialer Richtung elastisch deformierbar sind. Zum Festklemmen der Knochenplatte werden die Zungen radial zusammengepresst und in eine Bohrung oder Ansenkung in der Knochenplatte eingefügt, so dass die Knochenplatte durch das elastische radiale Ausfedern der Zungen am vorderen Ende des zweiten Spannelementes festgehalten wird. Ist die Knochenplatte dann an einem Knochen oder Knochenfragment festgeschraubt, kann das zweite Spannelement zurückgezogen werden, wodurch die elastischen Zungen aus der Ansenkung oder Bohrung in der Knochenplatte entfernt werden. Hergestellt sind die Zungen vorzugsweise durch parallel zur Längsachse vom vorderen Ende her in die hohlzylindrische Aussenwand des zweiten Spannelementes eingefräste Schlitze.

In einer anderen Ausführungsform der erfindungsgemässen Vorrichtung umfasst das Verbindungselement ein mit dem Stab fest verbundenes Verbindungsstück und eine Bohrung, worin das zweite Spannelement koaxial zur Längsachse verschiebbar gelagert ist. Die Spannmittel bestehen im wesentlichen aus einer konzentrisch zur Längsachse auf dem zweiten Spannelement angeordneten Klemmscheibe, einer ebenfalls konzentrisch zur Längsachse auf dem zweiten Spannelement angeordneten Rückstellfeder zur Rückführung der Klemmscheibe im unbelasteten Zustand und einem Sperrhebel zur Feststellung des zweiten Spannelementes relativ zum ersten Spannelement im angespannten Zustand der Vorrichtung. Zum Anspannen der Vorrichtung ist im Handgriff ein Bedienungshebel integriert, mittels welchem die Klemmscheibe beim Anspannen desselben auf dem zweiten Spannelement in eine verkantete Position bringbar ist und in der Folge bei weiterem Anspannen des Bedienungshebels das zweite Spannelement parallel zur Längsachse gegen das vordere Endstück des Bügels bewegbar ist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung die Reposition von Frakturen wegen des axialen Verschlussmechanismusses durch enge Zugänge im menschlichen Körper ermöglicht wird. Ein Führungsdraht resp. Kirschnerdraht, welcher durch das hohlzylindrische zweite Spannelement gesetzt werden kann, verhindert zusätzlich ein Abgleiten der Spannelemente an schrägen Knochenoberflächen. Der Führungsdraht resp. Kirschnerdraht kann soweit vorgetrieben werden, bis dieser den Knochen durchdringt. Danach wird der Führungsdraht resp. Kirschnerdraht in der Bohrung am vorderen Ende des ersten Spannelementes aufgenommen. Damit lässt sich eine starre Verstrebung der Vorrichtung am Knochen erreichen. Diese stabile Verbindung zwischen der erfindungsgemässen Vorrichtung und den Knochenfragmenten erlaubt eine kontrollierte Fragmentmanipulation.

Die Erfindung und Weiterbildungen der Erfindung werden im folgenden anhand der teilweise schematischen Darstellungen der Ausführungsbeispiele in den Figuren 7 bis 14 noch näher erläutert.

Es zeigen:
Fig. 1 eine Ansicht einer Vorrichtung, die nicht unter den Schutzbereich des Anspruchs 1 fällt;
Fig. 2 einen Schnitt durch das vordere Ende des ersten Spannelementes;
Fig. 3 einen Schnitt durch die bevorzugte Ausführungsform des zweiten Spannelementes;
Fig. 4 einen Schnitt durch eine weitere Ausführungsform des zweiten Spannelementes und eine an dessen vorderem Ende festgeklemmte Knochenplatte;
Fig. 5 die Anwendung der Vorrichtung an einer Beckenknochenfraktur;
Fig. 6 die Anwendung der Vorrichtung an einem Röhrenknochen;
Fig. 7 eine Ausführungsform der erfindungsgemässen Vorrichtung;
Fig. 8 eine weitere Ausführungsform des vorderen Endstückes des ersten Spannelementes mit einer Spannschraube;
Fig. 9 eine weitere Ausführungsform des vorderen Endstückes des ersten Spannelementes mit einem Einsatz und federnder Arretierung;
Fig. 10 eine weitere Ausführungsform des vorderen Endstückes des ersten Spannelementes mit einem Einsatz und Bajonettverschluss;
Fig. 11 eine weitere Ausführungsform der Mittel zur Anlage an einen Knochen;
Fig. 12 eine weitere Ausführungsform der Mittel zur Anlage an einen Knochen;
Fig. 13 eine weitere Ausführungsform der Mittel zur Anlage an einen Knochen mit einem Kugelgelenk; und
Fig. 14 eine weitere Ausführungsform der Mittel zur Anlage an einen Knochen.

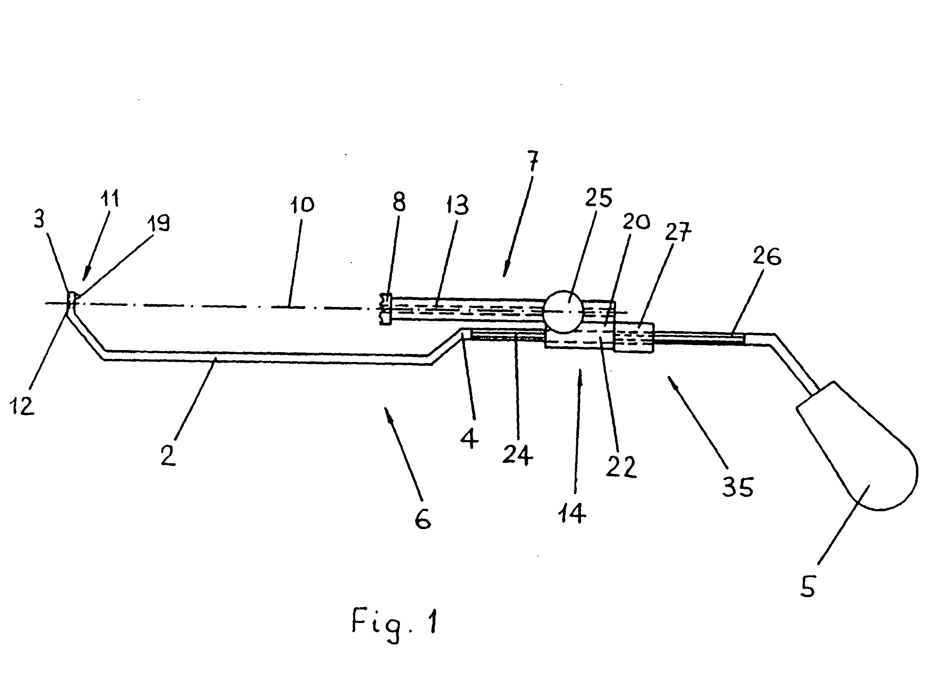

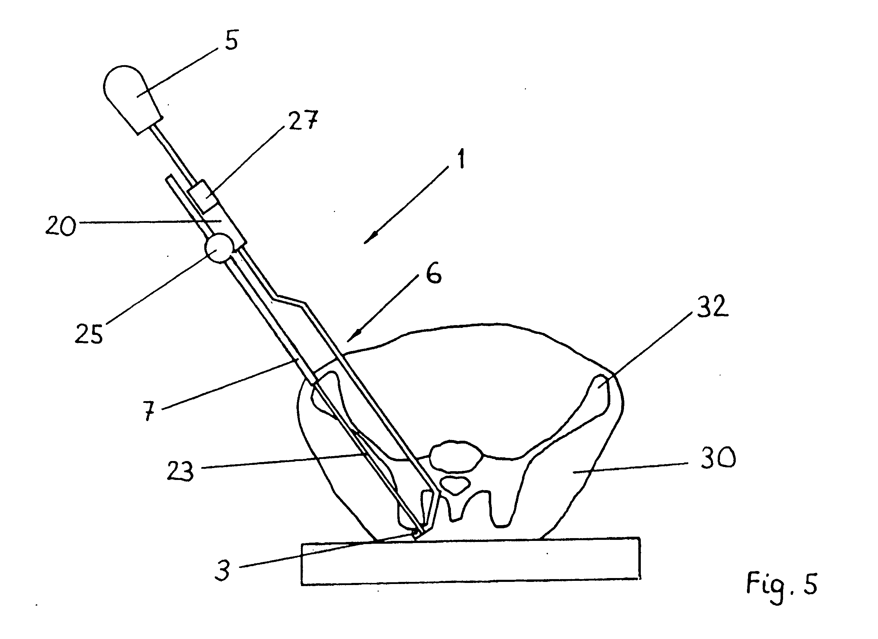

In Fig. 1 ist eine Vorrichtung zum Reponieren von Knochenfragmenten, insbesondere von Röhrenknochen oder dem Beckenknochen dargestellt. Die Vorrichtung umfasst ein erstes Spannelement 6, ein zweites Spannelement 7 und ein Verbindungselement 14 zur lösbaren Verbindung der beiden Spannelemente 6;7. Das erste Spannelement 6 umfasst einen Bügel 2, welcher ein vorderes Endstück 3, ein hinteres Endstück 4, sowie eine am vorderen Endstück 3 angeordnete, gegen das Innere des Bügels 2 gerichtete, kegelförmige Erhebung 11 aufweist, und aussen am Bügel 2 einen an dessen hinteres Endstück 4 anschliessenden zum ersten Endstück 3 senkrechten Stab 24 und einen Handgriff 5. Die Erhebung 11 dient zum Anlegen des Bügels 2 an ein Knochenfragment. Das zweite Spannelement 7 ist zylindrisch geformt und weist eine Längsachse 10 sowie ein vorderes Ende 8 auf, welches zur Anlage an ein weiteres Knochenfragment dient. Durch die Verbindungsmittel 14 sind die beiden Spannelement 6;7 so verbindbar, dass die Längsachse 10 das vordere Endstück 3 des Bügels 2 schneidet und das zweite Spannelement 7 parallel zur Längsachse 10 relativ zum ersten Spannelement 6 verschiebbar ist. Ferner weisen das vordere Endstück 3 und dass zweite Spannelement 7 konzentrisch zur Längsachse 10 Bohrungen 12;13 auf, welche die Aufnahme eines Führungsdrahtes resp. Kirschnerdrahtes 23 (Fig. 5) gestatten. Die Erhebung 11 weist eine Spitze 19 auf, welche in die Oberfläche eines Knochens einpressbar ist. Das Verbindungselement 14 besteht aus einer Muffe 20, welche mit dem zweiten Spannelement 7 verbunden ist und eine parallel zu dessen Längsachse 10 verlaufende und die Muffe 20 durchdringende Bohrung 22 zur verschiebbaren Aufnahme des Stabes 24 aufweist. Zudem umfasst das Verbindungselement 14 eine Feststellschraube 25, mittels welcher der Führungsdraht resp. Kirschnerdraht 23 (Fig. 5) in der Bohrung 13 des zweiten Spannelementes 7 lösbar blockierbar ist und eine Spannmutter 27, welche über das Aussengewinde 26 am Stab 24 schraubbar ist, so dass damit das Verbindungselement 14 mit dem zweiten Spannelement 7 parallel zur Zentralachse 23 gegen das vordere Endstück 3 des Bügels 2 pressbar sind.

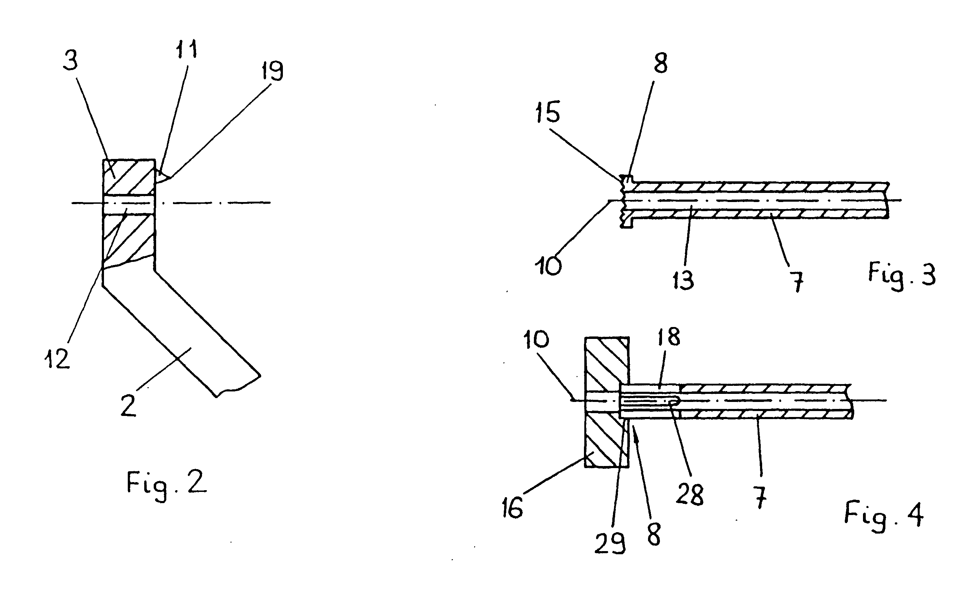

Fig. 2 zeigt die Erhebung 11 am vorderen Endstück 3 des Bügels 2. Die Erhebung 11 ist kegelförmig ausgestaltet und gegen das Innere des Bügels 2 gerichtet und neben der Bohrung 12 am vorderen Endstück 3 angeordnet.

In Fig. 3 ist das zweite Spannelement 7 mit einem kronenförmigen vorderen Ende 8 dargestellt. Dieses kronenförmige vordere Ende 8 ist mit stimseitigen Zacken 15, welche in die Oberfläche eines Knochens einpressbar sind, ausgebildet.

Fig. 4 zeigt eine Ausführungsform des zweiten Spannelementes 7 mit parallel zur Längsachse 10 verlaufenden Zungen 18 am vorderen Ende 8. Die Zungen 18 sind elastisch und radial deformierbar und dienen zum Festklemmen einer Knochenplatte 16. Dazu werden die Zungen 18 radial zusammengepresst und in eine Bohrung oder Ansenkung 29 in der Knochenplatte 16 eingefügt, so dass die Knochenplatte 16 durch das elastische radiale Ausfedern der Zungen 18 am vorderen Ende 8 des zweiten Spannelementes 7 festgehalten wird. Ist die Knochenplatte 16 dann an einem Knochen oder Knochenfragment festgeschraubt, kann das zweite Spannelement 7 zurückgezogen werden, wodurch die elastischen Zungen 18 aus der Ansenkung 29 oder Bohrung in der Knochenplatte 16 entfernt werden. Hergestellt sind die Zungen 18 durch parallel zur Längsachse 10 vom vorderen Ende 8 her in die hohlzylindrische Aussenwand des zweiten Spannelementes 7 eingefräste Schlitze 28.

Fig. 5 zeigt eine Vorrichtung angewendet bei einer Beckenfraktur, welche bei einem auf dem Rücken liegenden Patienten 30 horizontal verläuft. Durch einen ilio-inguinalen Zugang kann die Fraktur durch ungefähr senkrechtes Ziehen des entfernten unteren Fragmentes nach oben gegen das oben liegende Fragment reponiert werden. Die hier dargestellte Ausführungsform der Vorrichtung 1 wird mit der Erhebung 11 am vorderen Endstück 3 des ersten Spannelementes 6 an das untere Fragment des Beckenknochens angelegt und ausgerichtet. Danach wird das zweite Spannelement 7 mit dem Verbindungselement 14 soweit vorgeschoben, bis sein vorderes Ende 8 am oberen Knochenfragment anliegt, und anschliessend durch Anziehen der Spannmutter 27 die Fraktur des Beckenknochens zwischen dem ersten Spannelement 6 mit der Erhebung 11 und dem zweiten Spannelement 7 mit dem kronenförmigen vorderen Ende 8 komprimiert. Um ein Abgleiten der Erhebung 11 beziehungsweise des kronenförmigen vorderen Endes 8 an den schräggestellten Oberflächen des Knochens zu verhindern, kann mittels eines durch die Bohrung 13 (Fig. 1) im zweiten Spannelement 7 durchführbaren Bohrers eine Bohrung zur Aufnahme eines Führungsdrahtes resp. Kirschnerdrahtes 23 so weit durch den Beckenknochen gebohrt werden, dass der Führungsdraht resp. Kirschnerdraht 23 in der Bohrung 12 (Fig. 1 und 2) am vorderen 2) am vorderen Endstück 3 des ersten Spannelementes 6 aufgenommen wird. Der Führungsdraht resp. Kirschnerdraht 23 durchdringt nun den Knochen konzentrisch zur Längsachse 10 und wird am unteren Ende des Beckenknochens durch das vordere Endstück 3 des ersten Spannelementes 6 aufgenommen, während das zweite Spannelement 7 den Führungsdraht resp. Kirschnerdraht 23 am oberen Ende das Beckenknochens aufnimmt. Der Führungsdraht resp. Krischnerdraht 23 kann im zweiten Spannelement 7 mittels der Feststellschraube 25 lösbar blockiert werden. Somit entsteht eine starre Verstrebung der erfindungsgemässen Vorrichtung 1 am Beckenknochen. Mittels der Spannmutter 27 kann Druck auf beide Knochenfragmente ausgeübt und die Fraktur komprimiert werden.

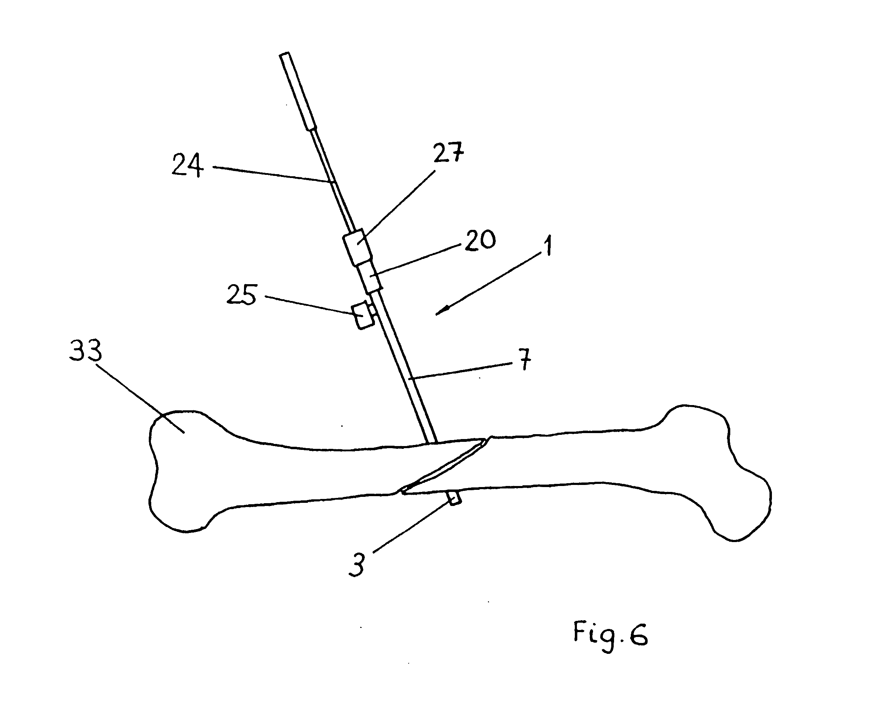

Fig. 6 zeigt eine weitere Anwendung der in den vorangehenden Figuren beschriebenen Ausführungsformen der Vorrichtung an einem langen Röhrenknochen 33. Grössere Fragmente von Röhrenknochen können knochennahe mit dem vorderen Endstück 3 des ersten Spannelementes 6 ventral oder dorsal umfahren und mit dem zweiten Spannelement 7 eingeklemmt werden. Sagital verlaufende Frakturen langer Röhrenknochen können somit über einen lateralen Zugang unter Kompression gebracht werden. Das Einbringen eines Führungsdrahtes resp. Kirschnerdrahtes 23 durch das zweite Spannelement 7 in ein Knochenfragment ohne Überbrücken der Fraktur gibt eine solide Verbindung zwischen Knochen und Vorrichtung im Sinne einer Hemicerclage. Dadurch wird die Manipulation des Fragmentes in allen 6 Freiheitsgraden möglich.

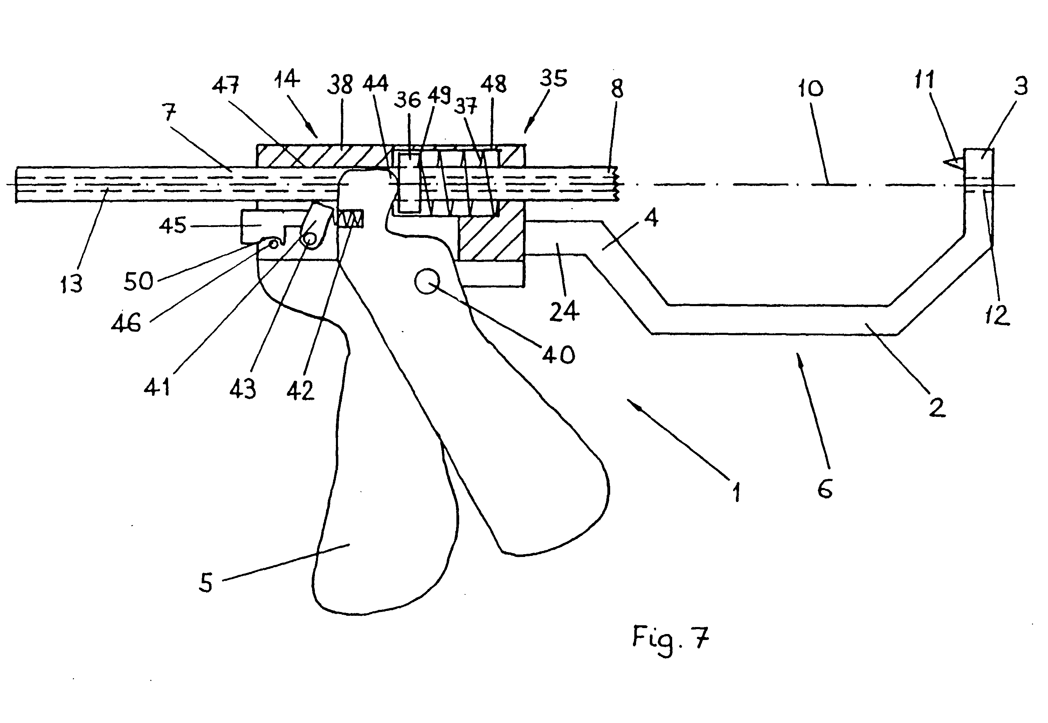

Fig. 7 zeigt eine Ausführungsform der erfindungsgemässen Vorrichtung. Die Vorrichtung 1 umfasst ebenfalls einen Bügel 2 mit einem an dessen hinteres Endstück 4 anschliessenden Stab 24, ein vorderes Endstück 3 mit einer gegen das innere des Bügels 2 gerichteten Erhebung 11. Der Bügel 2 wird beim Anbringen der Vorrichtung 1 um den Knochen herum geführt. Im weiteren enthält auch die hier beschriebene Ausführungsform der Vorrichtung 1 ein zweites Spannelement 7 mit einer Längsachse 10 und ein Verbindungselement 14 zur Verbindung des zweiten Spannelementes 7 mit dem Stab 24. Dieses Verbindungselement 14 besteht im wesentlichen aus einem plattenförmigen Verbindungsstück 38, welches am Stab 24 befestigt ist und eine dass Verbindungsstück 38 durchdringende Bohrung 47 zur Aufnahme des zweiten Spannelementes 7 enthält. Diese Bohrung 47 ist im Verbindungsstück 38 so ausgerichtet, dass die Längsachse 10 des zweiten Spannelementes 7 das vordere Endstück 3 des Bügels 2 schneidet und das zweite Spannelement 7 parallel zur Längsachse 10 relativ zum ersten Spannelement 6 verschiebbar ist. Ferner ist mit dem Verbindungsstück 38 ein Handgriff 5 lösbar verbunden. Am Handgriff 5 ist mittels einer ersten Achse 40 ein Bedienungshebel 39, welcher um die erste Achse 40 gegenüber dem Handgriff 5 schwenkbar ist, lösbar angebracht, so dass der Bedienungshebel 39 und der Handgriff 5 von der Vorrichtung 1 demontierbar sind. Als Spannmittel 35 dienen eine ringförmige Klemmscheibe 36 mit einer Bohrung 49 und ein Sperrhebel 41. Die Klemmscheibe 36 ist konzentrisch zur Längsachse 10 über das zweite Spannelement 7 geschoben, wobei die Bohrung 49 so bemessen ist, dass die Klemmscheibe 36 mit geringem Spiel auf dem zweiten Spannelement 7 axial verschiebbar ist. Zum Anspannen der Vorrichtung 1 wird der Bedienungshebel 39 gegen den Handgriff 5 gepresst, wodurch das klemmscheibenseitige Ende 44 des Bedienungshebels 39 exzentrisch auf die Klemmscheibe 36 drückt. Durch diese exzentrisch wirkende Druckkraft auf die Klemmscheibe 36 verkantet sich deren Bohrung 49 auf dem zweiten Spannelement 7 und schiebt dieses in der Folge in Richtung des vorderen Endstücks 3 des Bügels 2 bis die Spannkraft auf den Bedienungshebel 39 nachlässt. Wird keine Spannkraft auf den Bedienungshebel 39 ausgeübt, drückt die Rückstellfeder 37, welche ebenfalls konzentrisch zur Längsachse 10 angeordnet ist, in entgegengesetzter Richtung auf die Klemmscheibe 36 und schiebt in der Folge die Klemmscheibe 36 und den Bedienungshebel 39 in ihre hintere Ausgangslage zurück. Das zweite Spannelement 7 wird durch den Sperrhebel 41 in der gewünschten Position blockiert. Eine Feder 42 drückt den Sperrhebel 41 gegen die Mantelfläche des zweiten Spannelementes 7. Das zweite Spannelement 7 kann nur zurück geschoben werden, wenn dessen Blockierung durch den Sperrhebel 41 aufgehoben wird. Dies kann durch das Bedienen eines Entsperrungshebels 45 erreicht werden. Durch Drücken auf diesen Entsperrungshebel 45 wird der Sperrhebel 41 gegen die Feder 42 gepresst und die Berührung mit dem zweiten Spannelement 7 aufgehoben. Die Bewegung des Entsperrungshebels 45 wird durch einen Stift 46, welcher in einer entsprechenden Ausnahme 50 im Entsperrungshebel 45 angeordnet ist, begrenzt. Der Sperrhebel 41 ist um eine zweite Achse 43 drehbar gelagert und zusammen mit der Feder 42, dem Entsperrungshebel 45 und dem Stift 46 im Verbindungsstück 38 angebracht. Die Klemmscheibe 36 und die Rückstellfeder 37 sind in einem konzentrisch zur Bohrung 47 im Verbindungsstück 38 angeordneten Hohlraum 48 gelagert.

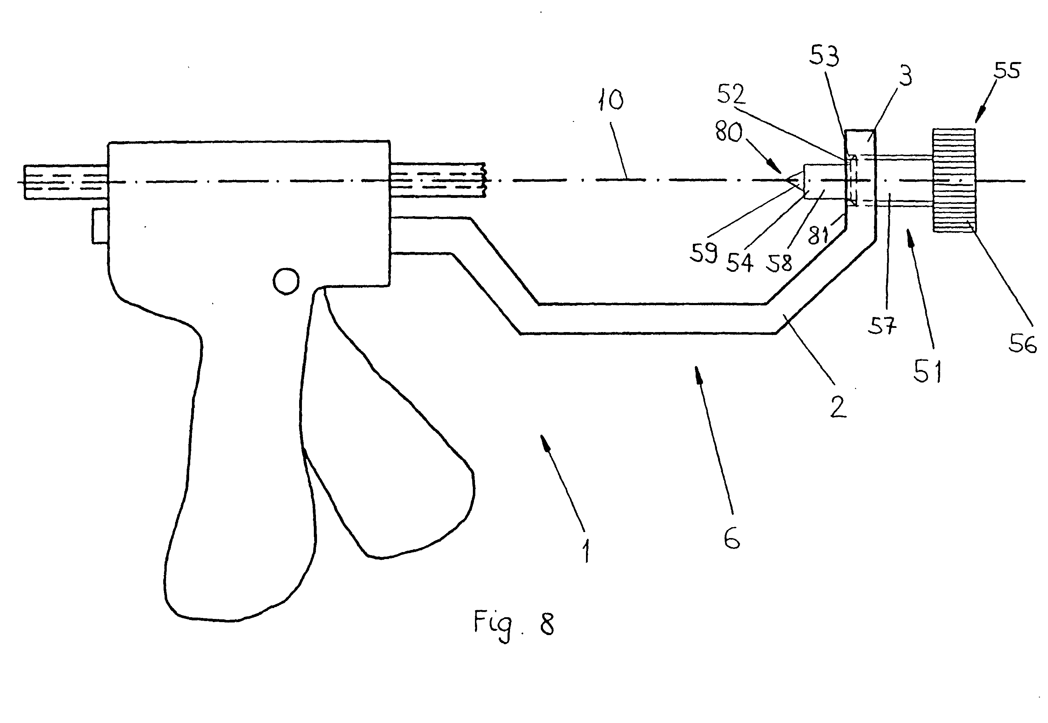

In Fig. 8 ist eine Ausführungsform des vorderen Endstückes 3 des ersten Spannelementes 6 gezeigt, welche sich von der in Fig. 7 dargestellten Ausführungsform nur darin unterscheidet, dass das vordere Endstück 3 konzentrisch zur Längsachse 10 eine Bohrung 52 mit einem Innengewinde 53 aufweist. Zusätzlich umfasst diese Ausführungsform der erfindungsgemässen Vorrichtung eine Spannschraube 51 mit einem vorderen, gegen die Innenseite 81 des Bügels 2 gerichteten Ende 54 und einem hinteren Ende 55. Die Spannschraube 51 umfasst vom hinteren Ende 55 her einen gerändelten Schraubenkopf 56, daran anschliessend einen Gewindeteil 57, dessen Gewinde mit dem Innengewinde 53 in der Bohrung 52 korrespondiert, und bis zum vorderen Ende 54 einen zylindrischen Schaftabschnitt 58. Die am vorderen Ende 54 angebrachten Mittel 80 zur Anlage an einen Knochen sind als konzentrisch zur Längsachse 10 angeordnete Spitze 59 ausgeführt. Mittels des Gewindeteils 57 ist die Spannschraube 51 konzentrisch zur Längsachse 10 in das vordere Endstück 3 einschraubbar, so dass beidseitig der Vorrichtung 1 axiale Verstellmöglichkeiten der an den Knochen anliegenden Teile der Vorrichtung 1 bestehen. Diese Ausführungsform des vorderen Endstückes 3 sowie der Einsatz einer solchen Spannschraube 51 ist auch an anderen Ausführungsformen der erfindungsgemässen Vorrichtung, beispielsweise der Ausführungsform gemäss Fig. 1 möglich.

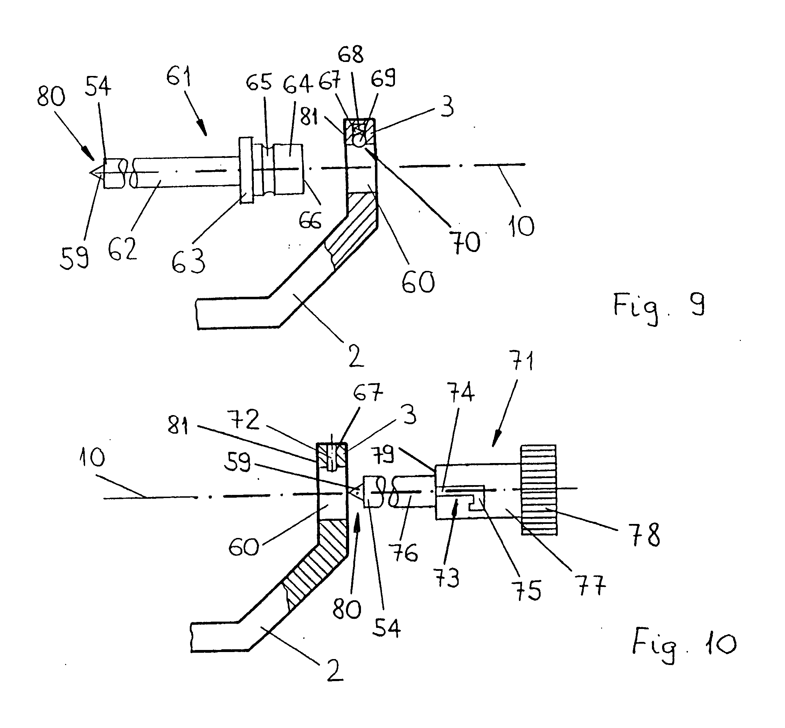

Fig. 9 zeigt eine weitere Ausführungsform des vorderen Endstückes 3 am Bügel 2. Das vordere Endstück 3 ist konzentrisch zur Längsachse 10 mit einer Bohrung 60 versehen. Ein zusätzlicher Einsatz 61 umfasst einen ersten Schaftabschnitt 62 mit dem gegen die Innenseite 81 des Bügels 2 gerichteten vorderen Ende 54 und einen zweiten zylindrischen Schaftabschnitt 64 am hinteren Ende 66 des Einsatzes 61. Der Schaftabschnitt 64 ist von der Innenseite des Bügels 2 her passend in die Bohrung 60 einführbar. Ein mittlerer Schaftabschnitt 63, welcher zwischen dem ersten Schaftabschnitt 62 und dem zweiten Schaftabschnitt 64 angeordnet ist und einen grösseren Durchmesser als der zweite Schaftabschnitt 64 aufweist, dient als axialer Anschlag der Einsatzes 61 gegen die Innenseite 81 des Bügels 2. Zur axialen Sicherung ist im vorderen Endstück 3 senkrecht zur Längsachse 10 eine Bohrung 67 angebracht, welche von der Bohrung 60 her in das vordere Endstück 3 eindringt, dieses aber nicht durchdringt. In der Bohrung 67 ist eine Feder 68 eingelegt, welche vom Boden der Bohrung 67 auf eine teilweise in die Bohrung 60 hineinragende Kugel 69 drückt. Die Kugel 69 kann beispielsweise durch Einpressen der in die Bohrung 60 mündenden Kanten der Bohrung 67 gegen ein Austreten aus der Bohrung 67 gesichert sein. Am Schaftabschnitt 64 ist konzentrisch und senkrecht zur Längsachse 10 eine Ringnute 65 mit kreissegmentförmigem Querschnitt angebracht. Beim Einfügen des Schaftabschnittes 64 in die Bohrung 60 schnappt die Kugel 69 durch die Federkraft in die Ringnute 65 ein und bietet eine axiale Sicherung des Einsatzes 61 in der Bohrung 60. Die am vorderen Ende 54 angebrachten Mittel 80 zur Anlage an einen Knochen sind als konzentrisch zur Längsachse 10 angeordnete Spitze 59 ausgeführt.

Fig. 10 zeigt eine weitere Ausführungsform des vorderen Endstückes 3 am Bügel 2. Das vordere Endstück 3 ist konzentrisch zur Längsachse 10 mit einer Bohrung 60 versehen. Zur axialen Sicherung ist im vorderen Endstück 3 senkrecht zur Längsachse 10 eine Bohrung 67 angebracht, welche von der Bohrung 60 her das vordere Endstück 3 durchdringt, und in welche ein Stift 72 eingesetzt ist. Ein zusätzlicher Einsatz 71 umfasst einen vorderen Schaftabschnitt 76 mit einem gegen die Innenseite 81 des Bügels 2 gerichteten vorderen Ende 54, einen mittleren Schaftabschnitt 77 und einen hinteren Schaftabschnitt 78. Der vordere und der mittlere Schaftabschnitt 76;77 sind von der Aussenseite des Bügels 2 koaxial in die Bohrung 60 einführbar. Der vordere Schaftabschnitt 76 sowie der mittlere Schaftabschnitt 77 sind kreiszylindrisch ausgestaltet, wobei der Durchmesser des vorderen Schaftabschnittes 76 geringer ist als derjenige des mittleren Schaftabschnittes 77. Am mittleren Schaftabschnitt 77 ist eine Nute 73 mit einem zur Längsachse 10 parallelen, ersten Teilstück 74 und einem daran anschliessenden zur Längsachse 10 senkrecht verlaufenden, zweiten Teilstück 75 angebracht. Das erste Teilstück 74 mündet in die an den vorderen Schaftabschnitt 76 grenzende Stirnfläche 79 des mittleren Schaftabschnittes 77, so dass die Nute 73 gegen den vorderen Schaftabschnitt 76 hin offen ist und beim Einschieben des Einsatzes 71 in die Bohrung 60 ein Eingreifen des Stiftes 72 in die Nute 73 ermöglicht. Ist der Einsatz 71 soweit in die Bohrung 60 eingeführt, dass der Stift in das zweite Teilstück 75 eingreifen kann, wird der Einsatz 71 um die Längsachse 10 verdreht und in der Bohrung 60 axial gesichert. Die am vorderen Ende 54 angebrachten Mittel 80 zur Anlage an einen Knochen sind als konzentrisch zur Längsachse 10 angeordnete Spitze 59 ausgeführt.

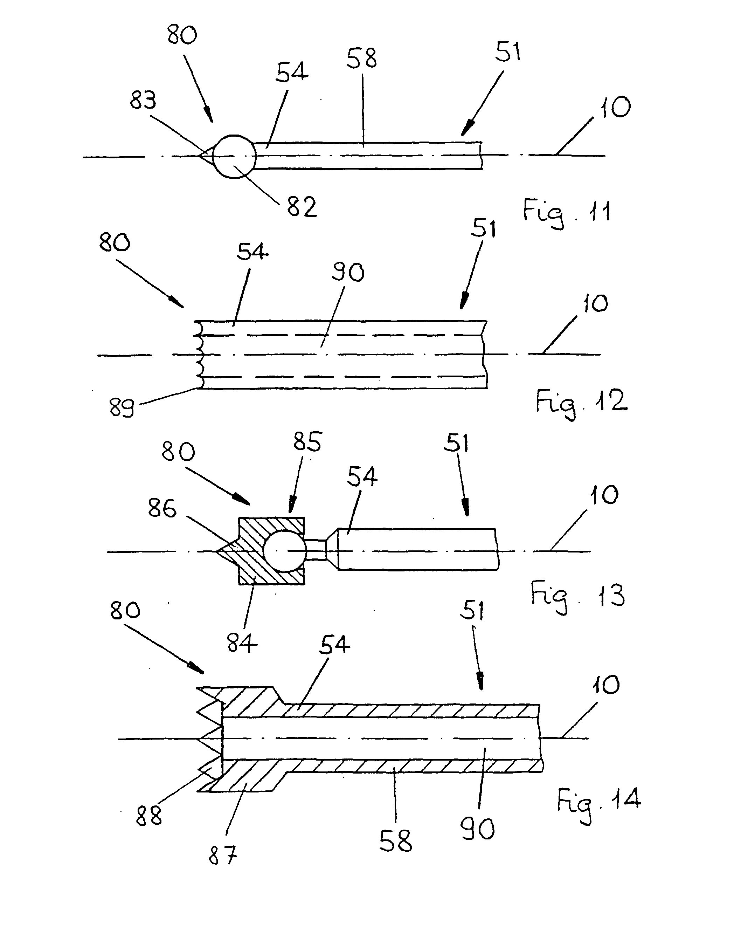

In den Fig. 11 bis 14 sind verschiedene Ausführungsformen der am vorderen Ende 54 der Spannschraube 51 angebrachten Mittel 80 zur Anlage an einen Knochen dargestellt. Bei der Variante gemäss Fig. 11 umfassen diese Mittel 80 eine zur Längsachse 10 konzentrische Kugel 82 mit einer kegelförmige Spitze 59. Der Durchmesser der Kugel 52 ist grösser als der Durchmesser des zylindrischen Schaftabschnittes 58 der Spannschraube 51. Damit ist der Vorteil erreichbar, dass die Spannschraube 51 auch ohne Trocar durch die Weichteile geschoben werden kann, ohne diese zu verletzten. Bei der in Fig. 13 dargestellten Variante umfassen die Mittel 80 einen Aufsatz 84 mit kegelförmiger Spitze 83 und ein Kugelgelenk 85, welches den den Aufsatz 84 mit dem vorderen Ende 54 der Spannschraube 51 verbindet. Durch diese Ausführungsform der Mittel 80 ist der Vorteil erreichbar, dass die Vorrichtung schräg auf die Knochenoberfläche aufgesetzt werden kann und die Spitze 83, welche in den Knochen eindringt, trotzdem senkrecht zur Knochenoberfläche steht. Die in den Fig. 12 und 14 dargestellten Varianten zeigen Mittel 80, welche stirnseitig mit zur Längsachse koaxiale, kronenartig angeordnete Zacken 88;89 umfassen. Bei der Variante gemäss Fig. 12 sind die Zacken 89 direkt am vorderen Ende 54 der Spannschraube 51 angebracht, während bei der Variante gemäss Fig. 14 am vorderen Ende 54 ein Ansatz 87 angebracht ist, dessen Durchmesser grösser als der Durchmesser des zylindrischen Schaftabschnittes 58 der Spannschraube 51 ist. Die Zacken 88 sind an der vom vorderen Ende 54 entfernten Stirnfläche des Ansatzes 87 angebracht. Der Vorteil der in den Fig. 12 und 14 dargestellten Varianten ist, dass die Spannschraube 51 mit einer zur Längsachse 10 koaxialen Bohrung 90 durchbohrt werden kann, worin je nach Durchmesser der Bohrung 90 ein Kirschnerdraht respektive Führungsdraht oder eine Schraube einführbar ist.

Die in den Fig. 11 bis 14 gezeigten Ausführungsformen der Mittel 80 sind beispielhaft an der Variante der Spannschraube 51 (Fig. 8) dargestellt und lassen sich in identischer Gestalt auf die Einsätze 61 (Fig. 9) und 71 (Fig. 10) übertragen. Auch ist eine Ausgestaltung des vorderen Endes 8 des zweiten Spannelementes 7 gemäss einer dieser Varianten möglich.

## Patentansprüche

1. Vorrichtung (1) zum Reponieren von Knochenfragmenten, insbesondere am Beckenknochen oder an Röhrenknochen mit
A) einem ersten Spannelement (6), welches ein vorderes Endstück (3) zur Anlage an ein Knochenfragment und ein hinteres Endstück (4) umfasst, und
B) einem zweiten Spannelement (7), welches ein vorderes Ende (8) zur Anlage an ein weiteres Knochenfragment umfasst; und
C) einem Verbindungselement (14), wodurch das erste und das zweite Spannelement (6;7) linear relativ zueinander verschiebbar verbunden sind, wobei
D) das erste Spannelement (6) zwischen dem vorderen Endstück (3) und dem hinteren Endstück (4) einen Bügel (2), welcher um einen Knochen oder ein Knochenfragment herum führbar ist, umfasst;
E) das zweite Spannelement (7) longitudinal ausgebildet ist und eine Längsachse (10) aufweist;
F) das Verbindungselement (14) so angeordnet ist, dass die Längsachse (10) des zweiten Spannelementes (7) das vordere Endstück (3) des ersten Spannelementes (6) im wesentlichen schneidet und das zweite Spannelement (7) parallel zur Längsachse (10) relativ zum ersten Spannelement (6) verschiebbar ist; und
G) die Vorrichtung (1) Spannmittel (35) umfasst, mittels welcher das zweite Spannelement (7) parallel zur Längsachse (10) gegen das vordere Endstück (3) des ersten Spannelementes (6) pressbar und relativ dazu lösbar feststellbar ist,
**dadurch gekennzeichnet, dass**
H) die Spannmittel (35) eine auf dem zweiten Spannelement (7) konzentrisch zur Längsachse (10) angeordnete Klemmscheibe (36) mit dazugehöriger Rückstellfeder (37) zur Rückführung der Klemmscheibe (36) im unbelasteten Zustand, sowie einen Sperrhebel (41) zur Feststellung dieses zweiten Spannelementes (7) relativ zum ersten Spannelement (6) im angespannten Zustand der Vorrichtung (1) umfassen.

2. Vorrichtung (1) nach Anspruch 1, **dadurch gekennzeichnet**, das erste Spannelement (6) einen an das hintere Endstück (4) anschliessenden Stab (24) umfasst.

3. Vorrichtung (1) nach Anspruch 1 oder 2, **dadurch gekennzeichnet, dass** das vordere Endstück (3) des Bügels (2) und dass zweite Spannelement (7) parallel zur Längsachse (10) Bohrungen (12;13) aufweisen, welche die Aufnahme eines Führungsdrahtes resp. Kirschnerdrahtes (23) gestatten.

4. Vorrichtung (1) nach Anspruch 1 oder 2, **dadurch gekennzeichnet, dass** das zweite Spannelement (7) parallel zur Längsachse (10) eine Bohrung (13) aufweist, welche die Aufnahme eines Trocars gestattet.

5. Vorrichtung (1) nach Anspruch 1 oder 2, **dadurch gekennzeichnet, dass** das zweite Spannelement (7) parallel zur Längsachse (10) eine Bohrung (13) aufweist, so dass eine Knochenschraube koaxial zur Längsachse (10) durchschiebbar und am vorderen Ende (8) in ein anliegendes Knochenfragment schraubbar ist.

6. Vorrichtung (1) nach einem der Ansprüche 1 bis 5, **dadurch gekennzeichnet, dass** das vordere Ende (8) des zweiten Spannelementes (7) kronenförmig mit stimseitigen Zacken (15), welche in die Oberfläche eines Knochens einpressbar sind, ausgebildet ist.

7. Vorrichtung (1) nach einem der Ansprüche 1 bis 5, **dadurch gekennzeichnet, dass** das vordere Ende (8) des zweiten Spannelementes (7) Mittel zum lösbaren Festklemmen einer Knochenplatte (16) umfasst.

8. Vorrichtung (1) nach Anspruch 7, **dadurch gekennzeichnet, dass** das vordere Ende (8) des zweiten Spannelementes (7) zur Längsachse (10) parallele, in radialer Richtung elastische Zungen (18) umfasst, welche ein lösbares Festklemmen einer Knochenplatte (16) ermöglichen.

9. Vorrichtung (1) nach einem der Ansprüche 1 bis 8, **dadurch gekennzeichnet, dass** der Bügel (2) eine am vorderen Endstück (3) angeordnete, gegen das Innere des Bügels (2) gerichtete Erhebung (11) mit einer Spitze (19) umfasst, welche in die Oberfläche eines anliegenden Knochenfragments einpressbar ist.

10. Vorrichtung (1) nach Anspruch 9, **dadurch gekennzeichnet, dass** die Erhebung (11) pyramiden- oder kegelförmig ist.

11. Vorrichtung (1) nach einem der Ansprüche 1 bis 10, **dadurch gekennzeichnet, dass** das Verbindungselement (14) ein mit dem hinteren Endstück (4) fest verbundenes Verbindungsstück (38) und eine Bohrung (47), worin das zweite Spannelement (7) parallel zur Längsachse (10) verschiebbar gelagert ist, umfasst.

12. Vorrichtung (1) nach einem der Ansprüche 1 bis 10, **dadurch gekennzeichnet, dass** das Verbindungselement (14) ein mit dem Stab (24) fest verbundenes Verbindungsstück (38) und eine Bohrung (47), worin das zweite Spannelement (7) parallel zur Längsachse (10) verschiebbar gelagert ist, umfasst.

13. Vorrichtung (1) nach Anspruch 11 oder 12, **dadurch gekennzeichnet, dass** ein Handgriff vorgesehen ist und dass im Handgriff (5) ein Bedienungshebel (39) integriert ist, mittels welchem die Klemmscheibe (36) beim Anspannen des Bedienungshebels (39) auf dem zweiten Spannelement (7) in eine verkantete Position bringbar ist und in der Folge bei weiterem Anspannen des Bedienungshebels (39) das zweite Spannelement (7) koaxial gegen das vordere Endstück (3) des Bügels (2) bewegbar ist.

14. Vorrichtung (1) nach Anspruch 1 oder 2, **dadurch gekennzeichnet, dass** das vordere Endstück (3) konzentrisch zur Längsachse (10) eine Bohrung (52) mit einem Innengewinde (53) und eine Spannschraube (51) mit einem Gewindeteil (57) umfasst, wobei das Gewinde des Gewindeteils (57) mit dem Innengewinde (53) korrespondiert, so dass die Spannschraube (51) koaxial zur Längsachse (10) in das vordere Endstück (3) einschraubbar ist.

15. Vorrichtung (1) nach Anspruch 1 oder 2, **dadurch gekennzeichnet, dass** das vordere Endstück (3) konzentrisch zur Längsachse (10) eine Bohrung (60), eine in die Bohrung (60) ragende, federnde Arretierung (70) und einen koaxial in die Bohrung (60) einschiebbaren Einsatz (61) mit einer konzentrisch und senkrecht zur Längsachse (10) ausgerichteten Ringnute (65) umfasst, so dass der Einsatz (61) durch Einschnappen der Arretierung (70) in die Ringnute (65) im vorderen Endstück (3) lösbar axial fixierbar ist.

16. Vorrichtung (1) nach Anspruch 1 oder 2, **dadurch gekennzeichnet, dass** das vordere Endstück (3) konzentrisch zur Längsachse (10) eine Bohrung (60), einen in die Bohrung (60) ragenden, radial angeordneten Stift (72) und einen koaxial in die Bohrung (60) einschiebbaren Einsatz (71) mit einer Nute (73) umfasst, wobei die Nute (73) auf einem ersten Teilstück (74) parallel zur Längsachse (10) und daran anschliessend auf einem zweiten Teilstück (75) senkrecht zur Längsachse (10) verläuft und zusammen mit dem Stift (72) einen Bajonettverschluss bildet, so dass der Einsatz (71) im vorderen Endstück (3) lösbar axial fixierbar ist.

17. Vorrichtung (1) nach Anspruch 14, **dadurch gekennzeichnet, dass** der Bügel (2) eine Innenseite (81) aufweist und die Spannschraube (51) ein gegen diese Innenseite (81) gerichtetes, vorderes Ende (54) mit Mitteln (80) zur Anlage an einen Knochen umfasst.

18. Vorrichtung (1) nach Anspruch 15 oder 16, **dadurch gekennzeichnet, dass** der Bügel (2) eine Innenseite (81) aufweist und der Einsatz (61;71) ein gegen diese Innenseite (81) gerichtetes, vorderes Ende (54) mit Mitteln (80) zur Anlage an einen Knochen umfasst.

19. Vorrichtung (1) nach Anspruch 17 oder 18, **dadurch gekennzeichnet, dass** die Mittel (80) aus einer zur Längsachse (10) konzentrischen, kegelförmigen Spitze (59) bestehen.

20. Vorrichtung (1) nach Anspruch 17 oder 18, **dadurch gekennzeichnet, dass** die Mittel (80) aus einer zur Längsachse (10) koaxialen, pyramidenförmigen Spitze bestehen.

21. Vorrichtung (1) nach Anspruch 17 oder 18, **dadurch gekennzeichnet, dass** die Mittel (80) aus einer zur Längsachse (10) koaxialen Kugel (82) mit einer zur Längsachse (10) konzentrischen, kegelförmigen Spitze (83) bestehen.

22. Vorrichtung (1) nach Anspruch 17 oder 18, **dadurch gekennzeichnet, dass** die Mittel (80) einen Aufsatz (84) mit kegelförmiger Spitze (83) und ein Kugelgelenk (85) umfassen, wobei das Kugelgelenk (85) den Aufsatz (84) mit dem vorderen Ende (54) verbindet.

23. Vorrichtung (1) nach Anspruch 17 oder 18, **dadurch gekennzeichnet, dass** die Mittel (80) aus zur Längsachse (10) koaxialen, kronenartig angeordneten Zacken (89) bestehen.

24. Vorrichtung (1) nach Anspruch 17 oder 18, **dadurch gekennzeichnet, dass** die Mittel (80) einen zur Längsachse (10) koaxialen Ansatz (87) mit kronenartig angeordneten Zacken (88) umfassen.

25. Vorrichtung (1) nach einem der Ansprüche 14, 23 oder 24, **dadurch gekennzeichnet, dass** die Spannschraube (51) mit einer zur Längsachse (10) koaxialen Bohrung (90) durchbohrt ist.

26. Vorrichtung (1) nach einem der Ansprüche 15 oder 16, **dadurch gekennzeichnet, dass** der Einsatz (61;71) mit einer zur Längsachse (10) koaxialen Bohrung (90) durchbohrt ist.

27. Vorrichtung (1) nach einem der Ansprüche 1 bis 26, **dadurch gekennzeichnet, dass** sie einen Handgriff (5) zum Halten der Vorrichtung (1) umfasst und dass der Handgriff (5) lösbar mit der Vorrichtung (1) verbunden ist.

## Claims

1. A device for repositioning bone fragments, particularly in the pelvic bone or in long bones, including
A) a first tension member (6) comprising a front end piece (3) to be brought to bear against a bone fragment and a rear end piece (4), and
B) a second tension member (7) comprising a front end (8) to be brought to bear against another bone fragment; and
C) a connecting member (14) by means of which the first and the second tension members (6;7) are connected with each other in such a way as to be rectilinearly displaceable relative to each other,whereby
D) the first tension member (6) comprises a bow (2) situated between the front end piece (3) and the rear end piece (4) which may be moved around a bone or a bone fragment;
E) the second tension member (7) is longitudinally shaped and has a longitudinal axis (10);
F) the connecting member (14) is arranged in such a way that the longitudinal axis (10) of the second tension member (7) essentially intersects the front end piece (3) of the first tension member (6) and that the second tension member (7) is displaceable relative to the first tension member (6) in a direction parallel to the longitudinal axis (10); and
G) the device comprises clamping means (35) by means of which the second tension member (7) may be pressed against the front end piece (3) of the first tension member (6) in a direction parallel to the longitudinal axis (10) and releasably locked relative thereto,
**characterised in that**
H) the clamping means (35) comprise a clamping disk (36) arranged concentrically to the longitudinal axis (10) on the second tension member (7), a readjusting spring (37) for pulling back the clamping disk (36) when in unstrained condition, as well as a catch lever (41) for locking this second tension member (7) relative to the first tension member (6) when the device (1) is in a strained condition.

2. A device as claimed in claim 1, **characterised in that** the first tension member (6) comprises a rod (24) attached to the rear end piece (4).

3. A device as claimed in claim 1 or 3, **characterised in that** the front end piece (3) of the bow (2) and the second tension member (7) include bores (12;13) extending parallel to the longitudinal axis (10) for receiving a guide wire or Kirschner wire (23).

4. A device as claimed in claim 1 or 2, **characterised in that** the second tension member (7) includes a bore (13) extending parallel to the longitudinal axis (10) which is apt for receiving a trocar.

5. A device as claimed in claim 1 or 2, **characterised in that** the second tension member (7) includes a bore (13) extending parallel to the longitudinal axis (10) so that a bone screw may be passed therethrough coaxially to the longitudinal axis (10) and, on the front end portion (8), be screwed into an adjacent bone fragment.

6. A device as claimed in any of the claims 1 to 5, **characterised in that** the front end portion (8) of the second tension member (7) is shaped in the form of a crown provided with frontal spikes which may be pressed into the surface of a bone.

7. A device as claimed in any of the claims 1 to 5, **characterised in that** the front end portion (8) of the second tension member (7) comprises means for releasably latching a bone plate (16).

8. A device as claimed in claim 7, **characterised in that** the front end portion (8) of the second tension member (7) comprises radially resilient arms (18) extending parallel to the longitudinal axis (10) which permit to releasably latch a bone plate (16).

9. A device as claimed in any of the claims 1 to 8, **characterised in that** on its front end piece (3) the bow (2) comprises a protrusion (11) directed towards the inside of the bow (2) and provided with a pointed tip (19) which may be pressed into the surface of an adjacent bone fragment.

10. A device as claimed in claim 9, **characterised in that** the protrusion (11) is of pyramidal or conical shape.

11. A device as claimed in any of the claims 1 to 10, **characterised in that** the connecting member (14) comprises a connecting piece (38) fixedly connected with the rear end piece (4) and a bore (47) wherein the second tension member (7) is mounted in such a way that it is displaceable coaxially to the longitudinal axis (10).

12. A device as claimed in any of the claims 1 to 10, **characterised in that** the connecting member (14) comprises a connecting piece (38) fixedly connected with the rear end piece (24) and a bore (47) wherein the second tension member (7) is mounted in such a way that it is displaceable coaxially to the longitudinal axis (10).

13. A device as claimed in claim 1 or 2, **characterised in that** a handle (5) is provided and that an operating lever (39) is integrated in the handle (5) by means of which, as the operating lever (39) is strained, the clamping disk (36) may be brought into a wedged position on the second tension member (7) and, while the operating lever (39) is maintained in its actuated position, the second tension member (7) is displaceable parallel to the longitudinal axis (10) towards the front end piece (3) of the bow (2).

14. A device as claimed in claim 1 or 2, **characterised in that** the front end piece (3) comprises a bore (52) with an internal screw thread (53) extending concentrically to the longitudinal axis (10) and a straining screw (51) with a threaded portion (57), the thread of the threaded portion (57) corresponding to the internal screw thread (53), so that the straining screw (51) may be screwed into the front end piece (3) coaxially to the longitudinal axis 10.

15. A device as claimed in claim 1 or 2, **characterised in that** the front end piece (3) comprises a bore (60) extending concentrically to the longitudinal axis (10), a resilient latch (70) extending into said bore (60), and an insert (61) insertable coaxially into said bore (60) and provided with an annular groove (65) extending concentrically and vertically to the longitudinal axis (10), so that by the engagement of the latch (70) with the annular groove (65) a releasable, axial fixation of the insert (61) in the front end piece (3) may be realised.

16. A device as claimed in claim 1 or 2, **characterised in that** the front end piece (3) comprises a bore (60) extending concentrically to the longitudinal axis (10), a peg (72) protruding radially into the bore (60), and an insert (71) coaxially insertable into the bore (60) and provided with a groove (73), said groove (73) having a first section (74) extending parallel to the longitudinal axis (10) continued by a second section (75) extending perpendicularly to the longitudinal axis (10), said groove and said peg (72) forming a bayonet locking so that a releasable, axial fixation of the insert (71) in the front end piece (3) may be realised.

17. A device as claimed in claim 14, **characterised in that** the bow (2) has an inside (81) and that the straining screw (51) comprises a front end portion (54) directed towards said inside (81) and including means (80) designed to bear against a bone.

18. A device as claimed in claim 15 or 16, **characterised in that** the bow (2) has an inside (81) and that the insert (61;71) comprises a front end portion (54) directed towards said inside (81) and including means (80) designed to bear against a bone.

19. A device as claimed in claim 17 or 18, **characterised in that** the means (80) consist of a conical tip (59) arranged concentrically to the longitudinal axis (10).

20. A device as claimed in claim 17 or 18, **characterised in that** the means (80) consist of a pyramidal tip arranged coaxially to the longitudinal axis (10).

21. A device as claimed in claim 17 or 18, **characterised in that** the means (80) consist of a ball (82) arranged coaxially to the longitudinal axis (10) and having a conical tip (83) arranged concentrically to the longitudinal axis (10).

22. A device as claimed in claim 17 or 18, **characterised in that** the means (80) comprise a top part (84) with a conical tip (83) and a ball-and-socket joint (85), the ball-and-socket joint (85) connecting the top part (84) with the front end portion (54).

23. A device as claimed in claim 17 or 18, **characterised in that** the means (80) consist of spikes (89) in a crown-like arrangement extending coaxially to the longitudinal axis (10).

24. A device as claimed in claim 17 or 18, **characterised in that** the means (80) consist of a shoulder (87) extending coaxially to the longitudinal axis (10) and provided with spikes (88) in a crown-like arrangement.

25. A device as claimed in any of the claims 13, 23 or 24, **characterised in that** the straining screw (51) is perforated by a bore (90) extending coaxially to the longitudinal axis (10).

26. A device as claimed in any of the claims 15 or 16, **characterised in that** the insert (61;71) is perforated by a bore (90) extending coaxially to the longitudinal axis (10).

27. A device as claimed in any of the claims 1 to 26, **characterised in that** the device comprises a handle (5) for holding the device and that the handle (5) is detachably connected to the device (1).

## Revendications

1. Dispositif (1) permettant de réduire des fragments d'os, notamment au niveau de l'os du bassin ou des os longs, comportant
A) un premier élément de serrage (6) qui comprend un bout avant (3) destiné à prendre appui sur un fragment d'os et un bout arrière (4), et
B) un deuxième élément de serrage (7) qui comprend une extrémité avant (8) destinée à prendre appui sur un autre fragment d'os; et
C) un élément d'assemblage (14) au moyen duquel le premier et le deuxième élément de serrage (6,7) sont reliés de manière à pouvoir coulisser linéairement l'un par rapport à l'autre,
D) le premier élément de serrage (6) comprenant, entre le bout avant (3) et le bout arrière (4), un archet (2) qui peut être passé autour d'un os ou d'un fragment d'os;
E) le deuxième élément de serrage (7) étant formé longitudinalement et présentant un axe longitudinal (10);
F) l'élément d'assemblage (14) étant disposé de telle sorte que l'axe longitudinal (10) du deuxième élément de serrage (7) coupe essentiellement le bout avant (3) du premier élément de serrage (6) et que le deuxième élément de serrage (7) peut coulisser par rapport au premier élément de serrage (6) parallèlement à l'axe longitudinal (10); et
G) le dispositif (1) comprenant des moyens de serrage (35), grâce auxquels le deuxième élément de serrage (7) peut être pressé parallèlement à l'axe longitudinal (10) contre le bout avant (3) du premier élément de serrage (6) et être bloqué de manière amovible par rapport à celui-ci,
**caractérisé en ce que**
H) les moyens de serrage (35) comprennent un disque de serrage (36) disposé de manière concentrique à l'axe longitudinal (10) et comportant un ressort de rappel (37) correspondant permettant de ramener le disque de serrage (36) lorsque celui-ci se trouve à l'état non chargé ainsi qu'un levier de blocage (41) permettant de bloquer ce deuxième élément de serrage (7) par rapport au premier élément de serrage (6) lorsque le dispositif (1) est à l'état tendu.

2. Dispositif (1) selon la revendication 1, **caractérisé en ce que** le premier élément de serrage (6) comprend une tige (24) située dans le prolongement du bout arrière (4).

3. Dispositif (1) selon la revendication 1 ou 2, **caractérisé en ce que** le bout avant (3) de l'archet (2) et le deuxième élément de serrage (7) présentent, parallèlement à l'axe longitudinal (10), des trous (12,13) qui permettent de recevoir un guide métallique ou fil de Kirschner (23).

4. Dispositif (1) selon la revendication 1 ou 2, **caractérisé en ce que** le deuxième élément de serrage (7) présente, parallèlement à l'axe longitudinal (10), un trou (13) qui permet de recevoir un trocart.

5. Dispositif (1) selon la revendication 1 ou 2, **caractérisé en ce que** le deuxième élément de serrage (7) présente, parallèlement à l'axe longitudinal (10), un trou (13), de sorte qu'une vis d'ostéosynthèse peut être introduite de manière coaxiale à l'axe longitudinal (10) et vissée à l'extrémité avant (8) dans un fragment d'os contigu.

6. Dispositif (1) selon l'une des revendications 1 à 5, **caractérisé en ce que** l'extrémité avant (8) du deuxième élément de serrage (7) est réalisé en forme de couronne avec des dents frontales (15) qui peuvent s'enfoncer dans la surface d'un os.

7. Dispositif (1) selon l'une des revendications 1 à 5, **caractérisé en ce que** l'extrémité avant (8) du deuxième élément de serrage (7) comprend des moyens permettant de bloquer de manière amovible une plaque d'ostéosynthèse (16).

8. Dispositif (1) selon la revendication 7, **caractérisé en ce que** l'extrémité avant (8) du deuxième élément de serrage (7) comprend des languettes (18) parallèles à l'axe longitudinal (10) et élastiques en sens radial qui permettent de bloquer de manière amovible une plaque d'ostéosynthèse (16).

9. Dispositif (1) selon l'une des revendications 1 à 8, **caractérisé en ce que** l'archet (2) comprend une saillie (11) disposée sur le bout avant (3) et orientée vers l'intérieur de l'archet (2), laquelle comporte une pointe (19) qui peut s'enfoncer dans la surface d'un fragment d'os contigu.

10. Dispositif (1) selon la revendication 9, **caractérisé en ce que** la saillie (11) est de forme conique ou pyramidale.

11. Dispositif (1) selon l'une des revendications 1 à 10, **caractérisé en ce que** l'élément d'assemblage (14) comprend une pièce de raccordement (38) reliée de manière fixe au bout arrière (4) et un trou (47) dans lequel est logé le deuxième élément de serrage (7) de manière à pouvoir coulisser parallèlement à l'axe longitudinal (10).

12. Dispositif (1) selon l'une des revendications 1 à 10, **caractérisé en ce que** l'élément d'assemblage (14) comprend une pièce de raccordement (38) reliée de manière fixe à la tige (24) et un trou (47) dans lequel est logé le deuxième élément de serrage (7) de manière à pouvoir coulisser parallèlement à l'axe longitudinal (10).

13. Dispositif (1) selon la revendication 11 ou 12, **caractérisé en ce qu'**une poignée est prévue et que dans la poignée (5) est intégré un levier de manoeuvre (39) qui, lorsque l'on tend ledit levier de manoeuvre (39), permet d'amener le disque de serrage (36) dans une position de blocage sur le deuxième élément de serrage (7), puis, en tendant d'avantage le levier de manoeuvre (39), de déplacer de manière coaxiale le deuxième élément de serrage (7) vers le bout avant (3) de l'archet (2).

14. Dispositif (1) selon la revendication 1 ou 2, **caractérisé en ce que** le bout avant (3) comprend, de manière concentrique à l'axe longitudinal (10), un trou (52) pourvu d'un filet intérieur (53) et une vis de serrage (51) pourvue d'une partie filetée (57), le filet de la partie filetée (57) correspondant au filet intérieur (53), de sorte que la vis de serrage (51) peut être vissée de manière coaxiale à l'axe longitudinal (10) dans le bout avant (3).

15. Dispositif (1) selon la revendication 1 ou 2, **caractérisé en ce que** le bout avant (3) comprend, de manière concentrique à l'axe longitudinal (10), un trou (60), un dispositif d'arrêt élastique (70) rentrant dans le trou (60) et un insert (61) pouvant être introduit de manière coaxiale dans le trou (60) et comportant une rainure annulaire (65) orientée de manière concentrique et perpendiculaire à l'axe longitudinal (10), de sorte que, grâce à l'enclenchement du dispositif d'arrêt (70) dans la rainure annulaire (65), l'insert (61) peut être fixé axialement de manière amovible dans le bout avant (3).

16. Dispositif (1) selon la revendication 1 ou 2, **caractérisé en ce que** le bout avant (3) comprend, de manière concentrique à l'axe longitudinal (10), un trou (60), une cheville (72) rentrant dans le trou (60) et disposée de manière radiale par rapport à celui-ci, ainsi qu'un insert (71) pouvant être introduit de manière coaxiale dans le trou (60) et comportant une rainure (73), la rainure (73) s'étendant sur une première section (74) parallèlement à l'axe longitudinal (10) et, sur une deuxième section (75), contiguë à celle-ci, perpendiculairement à l'axe longitudinal (10), et formant, ensemble avec la cheville (72), un dispositif de fermeture à baïonnette, de sorte que l'insert (71) peut être fixé axialement sur le bout avant (3) de manière amovible.

17. Dispositif (1) selon la revendication 14, **caractérisé en ce que** l'archet (2) présente une face intérieure (81) et **en ce que** la vis de serrage (51) comprend une extrémité avant (54) orientée vers ladite face intérieure (81) et comportant des moyens (80) destinés à prendre appui contre un os.

18. Dispositif (1) selon la revendication 15 ou 16, **caractérisé en ce que** l'archet (2) présente une face intérieure (81) et **en ce que** l'insert (61,71) comprend une extrémité avant (54) orientée vers ladite face intérieure (81) et comportant des moyens (80) destinés à prendre appui contre un os.

19. Dispositif (1) selon la revendication 17 ou 18, **caractérisé en ce que** les moyens (80) consistent en une pointe (59) de forme conique, concentrique à l'axe longitudinal (10).

20. Dispositif (1) selon la revendication 17 ou 18, **caractérisé en ce que** les moyens (80) consistent en une pointe de forme pyramidale, concentrique à l'axe longitudinal (10).

21. Dispositif (1) selon la revendication 17 ou 18, **caractérisé en ce que** les moyens (80) consistent en une sphère (82) coaxiale à l'axe longitudinal (10) comportant une pointe (83) de forme conique, concentrique à l'axe longitudinal (10).

22. Dispositif (1) selon la revendication 17 ou 18, **caractérisé en ce que** les moyens (80) comprennent un élément rapporté (84) comportant une pointe (83) de forme conique ainsi qu'une articulation à rotule (85), l'articulation à rotule (85) reliant l'élément rapporté (84) à l'extrémité avant (54).

23. Dispositif (1) selon la revendication 17 ou 18, **caractérisé en ce que** les moyens (80) se composent de dents (89) coaxiales à l'axe longitudinal (10), disposées en forme de couronne.

24. Dispositif (1) selon la revendication 17 ou 18, **caractérisé en ce que** les moyens (80) comprennent un embout (87) coaxial à l'axe longitudinal (10) comportant des dents (88) disposées en forme de couronne.

25. Dispositif (1) selon l'une des revendications 14, 23 ou 24, **caractérisé en ce que** la vis de serrage (51) est percée d'un trou (90) coaxial à l'axe longitudinal (10).

26. Dispositif (1) selon l'une des revendications 15 ou 16, **caractérisé en ce que** l'insert (61;71) est percé d'un trou (90) coaxial à l'axe longitudinal (10).

27. Dispositif (1) selon l'une des revendications 1 à 26, **caractérisé en ce que** celui-ci comprend une poignée (5) permettant de tenir le dispositif (1), et **en ce que** la poignée (5) est reliée de manière amovible au dispositif (1).
